(19) Europäisches Patentamt
European Patent Office
Office européen des brevets

(11) EP 4 310 102 A1

(12) # EUROPEAN PATENT APPLICATION

published in accordance with Art. 153(4) EPC

(43) Date of publication:
**24.01.2024 Bulletin 2024/04**

(21) Application number: **22770099.4**

(22) Date of filing: **15.03.2022**

(51) International Patent Classification (IPC):
***C07K 19/00*** *(2006.01)* ***A61K 39/00*** *(2006.01)*
***A61K 38/10*** *(2006.01)* ***A61K 38/17*** *(2006.01)*
***A61P 33/14*** *(2006.01)*

(52) Cooperative Patent Classification (CPC):
**A61K 38/10; A61K 38/17; A61K 39/00; C07K 19/00;** A61P 33/14

(86) International application number:
**PCT/BR2022/050087**

(87) International publication number:
**WO 2022/192974 (22.09.2022 Gazette 2022/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.03.2021 BR 102021004904**

(71) Applicants:
- **Biotick Pesquisa e Desenvolvimento Tecnológico Ltda**
  **04505-001 São Paulo-SP (BR)**
- **Empresa Brasileira De Pesquisa Agropecuária - EMBRAPA**
  **70770-901 Brasília - Df (BR)**

(72) Inventor: **ANDREOTTI E SILVA, Renato**
**70770-901 Brasília-DF (BR)**

(74) Representative: **Lavoix**
**Bayerstraße 83**
**80335 München (DE)**

Remarks:
The complete document including Reference Table(s) and the Sequence Listing(s) can be downloaded from the EPO website

(54) **PEPTIDE COMBINATION, CHIMERIC PEPTIDE, IMMUNOGENIC COMPOSITION, USE OF THE PEPTIDE COMBINATION, USE OF THE CHIMERIC PEPTIDE, USE OF A COMPOSITION, METHOD FOR INDUCING AN IMMUNE RESPONSE AND KIT**

(57) The present invention is applied to the field of Biotechnology and Immunology and refers to combinations of immunogenic peptides, chimeric peptides, compositions that comprise them and the uses thereof for control of ticks, particularly ticks of the species *Rhipicephalus microplus*. The present invention further provides kits containing the combinations, chimeric peptides and/or compositions comprising the combinations of immunogenic peptides, and a method for inducing immune response in animals, particularly bovines.

Daily engorged total
1600
1400
1200
1000
800
600
400
200
0
3/12
4/12
5/12
6/12
7/12
8/12
9/12
10/12
Group 1
Group 2
Control

FIGURE 2

EP 4 310 102 A1

## Description

## FIELD OF THE INVENTION

The present invention relates to the field of Biotechnology and Immunology. More specifically, the invention approaches the combinations of immunogenic peptides against mammal ticks, chimeric peptides, compositions that comprise them and the uses thereof.

The present invention further provides kits and a method of inducing immune response in mammal animals, particularly bovine.

## BACKGROUND OF THE INVENTION

Tick infestation can cause several problems in mammals, particularly for bovine cattle, affecting the health of the animals and reducing the production and quality of meat and milk.

As described by Blecha *et al.* (BLECHA, I.M.Z. et al. Analysis of Bm86 conserved epitopes: is a global vaccine against Cattle Tick Rhipicephalus microplus possible? Braz. J. Vet. Parasitol., Jaboticabal, v. 27, n. 3, p. 267-279 (2018), the tick *Rhipicephalus (Boophilus) microplus* is responsible for several losses in agribusiness, thus the control thereof is made mainly by means of chemical acaricides, which results in the contamination of products of animal origin and of the environment, apart from the fact that, the reports on the fact that the ticks appear to develop resistance to said acaricides become more and more frequent.

The resistance to several classes of acaricides is a problem that has been increasingly observed and reported, making researchers seek alternative measures for the control of tick infestation in bovines (AGUIRRE, A.A.R. et al. Design of the ATAQ peptide and its evaluation as an immunogen to develop a Rhipicephalus vaccine. Veterinary Parasitology.221 (2016) 30-38).

The search for effective combat against tick infestation in bovines is being carried out by different control methods, such as the chemical control (chemical compounds, such as arsenicals, organochlorines, formamidines, cyclomidines, pyrethroids, etc.) alternative control (natural predators, homeopathic products, pastures that would make it difficult for the larvae to climb, etc.) and immunologic control, which covers the use of different types of vaccines.

In particular, the technology on antigen vaccines against bovine ticks has been studied since the mid-90s, same time as the development and registration of the only commercially available vaccines against ectoparasites for the control of infestations by bovine ticks. The vaccine that is currently commercialized for this purpose, which contains the protein that is the homolog of the protein Bm86, presents variations in efficacy in the different geographic locations.

In this connection, Almazán et al. (ALMAZÁN, C. et al. Identification and characterization of Rhipicephalus (Boophilus) microplus candidate protective antigens for the control of cattle tick infestations. Parasitol Res. 106:471-479 (2010)) affirm that the recombinant antigen Bm86 of *Rhipicephalus (Boophilus) microplus* demonstrated protecting cattle against tick infestations. However, the variable efficacy of the vaccines based on Bm86 against geographic strains of ticks encouraged the development of research that seeks additional antigens for tick protection, to identify alternatives to the protein Bm86.

Some of these studies propose the use of complete proteins as antigens, such as trypsin inhibitors, subolesin, akirin, kallikrein, elastase inhibitors and peptides designed by reverse vaccinology in subsequent genomic studies.

Other lines of research propose the combination of antigens to obtain improved results in terms of efficacy in tick combat, considering that, in case a mechanism does not work or works in a less effective way, another mechanism can be duly efficient and exterminate the ticks by causing irreversible damage to some of their vital metabolic functions.

Despite the increase in the scientific knowledge about the role of these proteins in the control of bovine ticks, the alternative approaches to Bm86 described up to now have not shown to be promising enough for the manufacture of a commercial product, thus, improved strategies, more efficient and/or immunization agents, particularly for large scale vaccination, are still necessary.

Consequently, there still exists the need for a more efficient vaccine against ticks in mammal animals, particularly bovine cattle for the control of ticks of the species *Rhipicephalus (Boophilus) microplus.* Therefore, it is an objective of the present invention to overcome the disadvantages of the state of the art and supply this need by providing a peptide combination and composition able to reach unexpected and efficient results against tick infestation.

## BRIEF DESCRIPTION OF SEQUENCES

The biologic sequences used for the development of the present invention, are described in the section Sequence Listing.

**SEQ ID NO: 1** relates to an artificial sequence that is similar to a peptide fragment which function is related to the formation of the insect cuticle protein. Presents 73% sequence identity with *I. scapularis.*

**SEQ ID NO: 2** relates to an artificial sequence that is similar to a peptide fragment which function is related to the formation of the insect cuticle protein. Presents 67% sequence identity with *I. scapularis.*

**SEQ ID NO: 3** relates to an artificial sequence that is similar to a peptide fragment which function is related to the formation of the embryo. Presents 88% sequence identity with *I. scapularis.*

**SEQ ID NO: 4** relates to an artificial sequence that is similar to a peptide fragment which function is related to the formation of the embryonated eggs. Presents 70% sequence identity with *I. scapularis.*

**SEQ ID NO: 5** relates to an artificial sequence that is similar to a peptide fragment which function is related to the formation of the embryonated eggs. Presents 55% sequence identity with *I. scapularis.*

**SEQ ID NO: 6** relates to an artificial sequence that is similar to a peptide fragment which function is related to the mechanisms of signal transduction (TPR). Presents 90% sequence identity with *I. scapularis.*

**SEQ ID NO: 7** relates to an artificial sequence that is similar to a peptide fragment which function is related to the formation of the zinc-dependent metalloprotease. Presents 30% sequence identity with *R.microplus.*

**SEQ ID NO: 8** relates to an artificial sequence that is similar to a peptide fragment which function is related to the kazal-type serine protease inhibitor. Presents 45% sequence identity with *I. scapularis.*

**SEQ ID NO: 9** relates to an artificial sequence that is similar to a peptide fragment which function is related to the chitin connection. Presents 67% sequence identity with *I. scapularis.*

**SEQ ID NO: 10** relates to an artificial sequence that is similar to a peptide fragment which function is related to the trypsin inhibitor domain (Kunitz). Presents 43% sequence identity with *R. microplus.*

**SEQ ID NO: 11** relates to an artificial sequence that is similar to a peptide fragment which function is related to the trypsin inhibitor (BPTI) - Kunitz. Presents 43% sequence identity with *R. microplus.*

**SEQ ID NO: 12** relates to an artificial sequence that is similar to a peptide fragment which function is related to the saliva molecules that modulate host response. Presents 35% sequence identity with *I. scapularis.*

## SUMMARY OF THE INVENTION

The present invention aims at solving the inconveniences of the state of the art cited above by means of new peptide combinations that are able to present satisfactory immunogenic response against ticks in mammals, particularly bovine cattle, particularly ticks of the species *Rhipicephalus microplus.* The new combinations of specific peptides of the present invention are able to reach greater technical advantages as regards the suppression of the vital metabolic functions of the referred ticks.

Thus, in a first aspect, the present invention refers to a combination of at least 2 immunogenic peptides selected from the group consisting in the peptides of SEQ ID NO: 1 to 12.

In a second aspect, the present invention refers to a chimeric peptide comprising combinations of at least 2 immunogenic peptides selected from the group consisting in the peptides of SEQ ID NO:1 to SEQ ID NO: 12, connected to each other by any means known to the state of the art.

In a third aspect, the present invention refers to an immunogenic composition comprising the combination of peptides or the chimeric peptide of the present invention and pharmaceutically acceptable excipients.

The present invention further refers to the use of the combination of peptides, the chimeric peptide or the composition described herein, to immunize, treat, protect, attenuate and/or prevent tick infestations in a mammal animal, particularly bovine cattle.

In a further aspect, the present invention refers to a method to induce immune response in mammal animals, particularly bovine cattle, wherein the method comprises obtaining the composition containing the chimeric peptide or the immunogenic composition herein described the administration thereof to an animal in need thereof and monitoring of the degree of tick infestation in the referred animal.

In a further aspect, the present invention refers to a kit, comprising the peptide combination, the chimeric peptide or the immunogenic composition, as defined by the present invention. Preferably, the kits are for vaccination.

Any of the objects and processes described above can serve as basis to compose the other objects and the preferred embodiments thereof, even in case said relation(s) has(have) not been explicitly described.

## BRIEF DESCRIPTION OF THE FIGURES

Figure **1** illustrates, as state of the art, the biological cycle of the cattle tick.

**Figure 2** illustrates the graph with the average amount of ticks (engorged) collected daily by group during the tick drop period in the infested animals.

**Figure 3** illustrates the total number of ticks collected in the groups tested after the challenge.

**Figure 4** illustrates the average weight of the ticks (engorged) and the respective laying per group.

**Figure 5** illustrates the average immune response (IgG) of the animals vaccinated with peptides in the groups tested. All the egg samples were incubated until hatched and the hatchability rate was evaluated in the groups for the purposes of calculation of the efficacy of the immunoprotection of the bovines. In Figure 5 we can observe the IgG production in groups 1 and 2 which corresponds to the immune response of the animals in face of the peptide stimulation. The results were observed in the ELISA method.

## DETAILED DESCRIPTION OF THE INVENTION

Unless otherwise defined, all the technical expressions, annotations and other scientific terminology used herein are destined to comprise the meanings usually understood by those skilled in the field of the present invention. In some cases, the expressions with commonly understood meanings are defined in the present document with the purpose of bringing clearness and/or for ready reference, and the inclusion of said definitions in the present document must not be interpreted, necessarily, as representing a substantial difference in relation to what is generally understood in the state of the art.

The techniques and procedures described herein or referred by the present document are usually well understood and employed using the conventional methodology by those skilled in the art. As appropriate, the processes involving the use of kits and commercially available reagents are usually carried out in accordance with protocols and/or parameters defined by the manufacturer, save when otherwise indicated.

It must be emphasized that the present invention, where appropriate, is not limited to the methodology, protocols, cell lines, genus or species of animals, constructions and specific reagents described, which, in an obvious manner, can vary. Additionally, the terminology used in the present document has only the purpose of describing examples of specific embodiments and is not destined to limit the scope of the present invention.

Throughout this document, the single forms "one" and "the", or single forms of any expression or term, include the references to the plural, unless the context clearly informs otherwise.

Throughout this document, the expression "comprises", and any variations such as "the comprise" or "comprising", must be interpreted as being "open expressions" able to imply the inclusion of additional elements or groups of elements, which were not explicitly described, not having limiting character.

Throughout this document, the expression "consists", and any variations such as "consist" or "consisting", must be interpreted as "closed expressions", not implying the inclusion of additional elements or groups of elements, which were not explicitly described, having limiting character.

Throughout this document, the exact values or ranges of exact values provided in relation to a factor, quantity, concentration or particular preference must be interpreted as also providing approximate values or ranges of values, such as by means of the expression "around".

Throughout this document, words and expressions such as "preferably", "particularly", "as", "such as", "more particularly" and similar, and variations thereof, must be interpreted as being entirely optional characteristics, preferred embodiments or possible non-exhaustive examples, without conferring scope limiting character.

Throughout this document, all the tiles and subtitles are used solely for convenience and must not be interpreted as being limitations of the present invention.

Throughout this document, the word "peptide", must be interpreted as being a single linear chain of monomer amino acids linked by peptide bonds. Amino acids which have been incorporated in peptides are called "residues". Every peptide has an N-terminal residue and a C-terminal residue in the corresponding extremities of the peptide. Further, throughout this document, the word "peptide" must be interpreted as comprising any number of consecutive residues. Further, the expressions "peptide", "polypeptide" and "protein" must be interpreted as synonyms, being interchangeable with each other and must not be interpreted as having a determined number of residues.

Throughout this document, the expression "chimeric peptide" must be interpreted as a peptide originating from the junction, bond, union or connection of two or more equal or different peptide fragments, as defined by the present invention, in any order, wherein said junction, bond, union or connection between peptide fragments can be performed by any means known to the state of the art. In a preferred embodiment, said junction, bond, union or connection between the peptide fragments is performed by a spacer. The peptide-spacer link is preferably performed by means of a peptide bond.

Further, throughout this document, the expressions "peptide", "chimeric peptide", "polypeptide", "peptide fragment" and "protein" must be interpreted as having any shape, being linear or having native three-dimensional structure or of particular interest. The peptide fragments to be bonded may originate from microorganisms of the same species or different species. Further, the peptide fragments and the chimeric peptides can be synthetically obtained (for example, by solid phase synthesis techniques), originated in recombinant form (for example, by means of use of expression cassette under the control of a specific promoter) or from any pertinent technologies of the field of the invention known by a person skilled in the art. Sufficient techniques for guidance are also found in the literature.

According to the present invention, structural modifications to improve the stability *in vivo* of the peptides, as well as improve the molecular properties related to the action mechanism, resulting in improvement of the pharmacologic activity (peptidemimetics), if any, must be considered as within the scope of the present invention.

Said modifications can be limited specifically to protect or replace the labile bond (peptide bond), from the introduction of fragments/atypical portions (non-peptide), or even alter the peptide conformation. Promising molecular modifications (non-peptide) would be those that attempt to mimetize the structure and molecular properties of the peptide.

Examples of possible molecular modifications would be: pseudopeptides, which present structural modifications in the main chain of at least one peptide bond with isoster groups or iso-electronic, such as reduced amides, azopeptides, retro-inverse peptides, peptoids; replacement with unnatural amino acids, such as replacement of L-amino acids with respective D-enantiomers; use of N-alkyl amino acids, alpha-amino acids, alpha-substituted, alpha amino acids beta-substituted, proline analogues, gama- and beta-amino acids; ether bridges, biaryl, disulfide or others that mimetize those already cited; N and C terminal connection with lateral chains of amino acids (Gentilucci, L., De Marco, R., Cerisoli, L. Chemical Modifications Designed to Improve Peptide Stability: Incorporation of Non-Natural Amino Acids, Pseudo-Peptide Bonds, and Cyclization. Current Pharmaceutical Design, v. 16, p. 3185-3203, 2010).

Additionally, a person skilled in the art would recognize that, conservative replacements that result in alteration of an amino acid by another chemically similar amino acid in a sequence, are within the scope of the present invention. Said conservative substitutions providing similarly functional amino acids are known in the art.

In one embodiment, the present invention refers to a combination of at least 2 immunogenic peptides selected from the group consisting in the peptides of SEQ ID NO: 1 to 12.

In an additional embodiment, the present invention refers to a chimeric peptide comprising combinations of at least 2 immunogenic peptides selected from the group consisting in the peptides of SEQ ID NO: 1 to SEQ ID NO: 12. The peptide fragments in the chimeric peptide can be linked to each other by any means known to the state of the art. In a preferred embodiment, said peptide fragments are linked to each other by means of a spacer.

The expression "peptide combination" must be interpreted as being a grouping, collection or aggregate comprising at least two peptides of SEQ ID NO:1 to 12 as defined in the present invention. In this context, the expression "at least two" refers to the quantity from 2 to 24 peptides, as well as any range between said values. Preferably, the peptide combination, according to the present invention, comprises from 2 to 8 peptides, preferably from 3 to 7 peptides, more preferably from 4 to 6 peptides.

In a preferred embodiment, the combination or the chimeric peptide of the present invention comprises 6 peptides selected from the group consisting in the peptides of SEQ ID NO: 1 to 12. In particular, the combination or the chimeric peptide of the present invention comprises the peptides of SEQID NOs: 1 to 6 or the peptides of SEQ ID NOs: 7 to 12.

As previously pointed out, the peptide fragments that compose the chimeric peptide, object of the present invention, can be linked to each other by any means or way known to the state of the art. In a preferred embodiment, said fragments are linked to each other by means of a spacer. In this context, as previously pointed out, "spacer" must be interpreted as being a junction, bond, union or connection of the peptides, as defined in the present invention. For example, the spacer may be constituted by one or more glycine residues (Gly or G) or one or more proline residues (Pro or P) or any combinations thereof. For example, the spacer can be GPGPG. Preferably, the spacer is constituted by one or more glycine residues. More preferably, the spacer is constituted by 2 to 15 glycine residues, particularly 2 to 10 glycine residues, more particularly 3 to 5 glycine residues. Said glycine residues were selected to confer conformation freedom (flexibility) to the resulting hybrid peptide, without compromising the action predicted to a great extent. Thus, the nature of the spacer is not particularly relevant or critical for the present invention. In this sense, it must be understood, according to the present invention that any spacer that grants an equivalent function to the resulting chimeric peptide, is within the scope of the present invention.

In another embodiment, the present invention refers to an immunogenic composition comprising the peptide combination or the chimeric peptide of the present invention and pharmaceutically acceptable excipients, in therapeutically efficient amounts.

The expression "pharmaceutically acceptable excipient" must be interpreted as being any vehicle, or substance which is not a pharmaceutically active ingredient. Therefore, it must be understood, according to the present invention, that any conventional pharmaceutically acceptable excipient, in any form, usually employed to act as excipient, is within the scope of the present invention. Pharmaceutically acceptable excipients are duly known by those skilled in the art, and they may be chosen from Remington: The Science and Practice of Pharmacy, Remington, 22nd edition or Handbook of pharmaceutical excipients, Rowe, 8th edition, herein incorporated as reference. The pharmaceutically acceptable excipient includes but is not limited to: buffer or saline solution, stabilizer, surfactant, solubilizer, diluents, carriers, emulsifier and preservative.

Preferably, the excipient is added in an amount from around 1 to 3 ml per dose. More preferably, 2 ml per dose.

Throughout the present document, the expression "diluent" must be interpreted as being any substance having the function of diluting, conserving the properties of what is being diluted. Therefore, it must be understood, according to the present invention, that any conventional diluent, in any form, usually employed to dilute, is within the scope of the present invention. Acceptable diluents are duly known by a person skilled in the art. The diluent includes, but is not limited to: water, alcohol and sterile solutions.

Throughout the present document, the expression "adjuvant" must be interpreted as being any substance that modulates or increases the immunogenicity of the peptides of the present invention. Therefore, it must be understood, according to the present invention, that any conventional adjuvant, in any form, usually employed in compositions with this purpose, is within the scope of the present invention. The adjuvant includes, but its not limited to: Montanide®, Freund complete adjuvant, Freund incomplete adjuvant, aluminum hydroxide, aluminum phosphate, silica and saponin.

Throughout the present document, the expression "carrier" must be interpreted as being a transporter, permease, or any structure wherein the peptide can be incorporated or with which it may be associated, to guide and/or ease and/or improve the triggering of an immune response by a human being or an animal. Therefore, it must be understood, according to the present invention, that any conventional carried, in any form, usually employed to act as carrier is within the scope of the present invention. The carrier includes but is not limited to: any inert heterologous substance, KLH, horse albumin, colloidal gold particles, antibodies or fragments of antibodies, polymers, vesicles and nanovesicles.

By "stabilizer agents" it must be understood, but not limited to, saccharides, trehalose, and mannitol, sucrose and similar, to increase and/or maintain the shelf life of the product and/or to enhance the stability, or combinations thereof.

In a preferred embodiment, the composition of the present invention can further comprise an additional biologically active substance or compound.

Throughout the present document, the expression "additional biologically active substance or compound" must be understood as being any substance or compound that exerts a biologically active effect. Therefore, it must be understood, according to the present invention, that any additional biologically active substance or compound, in any form, usually employed to act as such, is within the scope of the present invention. They can exert their function by the same mechanism or by different or similar mechanisms. They can have a multiplicity of related action mechanisms and/or have independent action mechanisms. The additional biologically active substances or compounds include, but are not limited to: enhancers, agonists, etc.

Throughout the present document, the expression "therapeutically effective amount" refers to the quantity of peptide that suffices to result in a therapeutic effect in the animal. The therapeutically effective amount can be promptly determined by a person skilled in the art following routine procedures, without undue experimentation, considering the route of administration, the specific composition used, the clinical factors, the age and weight of the human or animal, among others, if that is the case, as to be independent of the cited factors. Preferably, the therapeutically efficient amount is from 50 to 500 $\mu$g, more preferably from 100 to 300 $\mu$g, more preferably of 200 $\mu$g.

The composition of the present invention can be formulated according to the standard methodology known by a person skilled in the art, not being particularly relevant to the present invention. Further, the composition of the present invention can be in any form, considering the function of the composition, the route of administration, and the desired effect. Possible forms include, but are not limited to: solution, mixture, powder, granules, aerosol, or freeze dried.

The composition of the present invention can be administered to the animal by various standard routes of administration, considering the form of the composition and the desired effect. It can be for a mainly local effect, or systemic. The composition of the present invention can, for example, be in the form of a solution for subcutaneous administration, in aerosol form for intra-nasal administration or even in the form of a solution for intramuscular administration, for example, in the neck plate region. Other routes of administration include, but are not limited to: oral, topical, implantable, intravenous, intra-arterial, intradermal, intra-peritoneal and parenteral.

The composition of the present invention is preferably a vaccine. Preferably, the composition form of the vaccine of the present invention is a solution, for subcutaneous administration.

By "immunogenic composition" it must be understood that it relates to a composition comprising at least one antigen that provokes an immunologic response in the host of an immune cell response and/or mediated by host to immunogenic composition or applied vaccine. Preferably, the host will exhibit a therapeutic or protective immunologic response, so that the resistance to a new infestation will be improved and/or the clinical severity of the disease will be reduced.

The expression "vaccine" is destined to the use of an immunologically effective amount of one or more immunogenic compound(s) and a pharmaceutically acceptable vehicle. The immunogenic compounds for the present invention are the peptides of SEQ ID NO: 1 to SEQ ID NO: 12.

The composition of the present invention is preferably for immunizing, treating, protecting, attenuating and/or preventing tick infestation in mammal animals, particularly bovine cattle animals. In a preferred embodiment, the tick is *Rhipicephalus microplus.*

The antigenic composition or vaccine described herein can be administered in the most appropriate or necessary dose to provide an immune response of interest in the animal, particularly the bovine.

In one embodiment, the present invention refers to the use of peptide combinations, of the chimeric peptide, or the composition described herein, to prepare a composition or medication to immunize, treat, protect, attenuate and/or prevent tick infestations in mammal animals, particularly bovine cattle animals, by means of the immunization of these animals.

In one embodiment, the peptide combination, chimeric peptide or immunogenic composition is administered to the animals in a concentration from 50 to 500 μg, more preferably from 100 to 300 μg, even more preferably of 200 μg. In a preferred embodiment, there are administered 3 doses in an interval varying from 10 to 30 days, preferably 21 days.

The "sequence identity" as it is known in the state of the art, refers to a relation between two or more sequences of polypeptides or two or more sequences of polynucleotides, that is, a reference sequence and a sequence given to be compared with the reference sequence. The identity of the sequence is determined comparing the given sequence to the reference sequence after the sequences have been ideally aligned to produce the highest level of similarity of sequence, as determined by the correspondence between character chains of said sequences. During said alignment, the sequence identity is determined on a position-by-position basis, for example, the sequences are "identical" in a particular position if in this position the nucleotides or amino acid residues are identical.

The total number of said position identities is, subsequently, divided by the total number of nucleotides or residues in the reference sequence to provide % of sequence identity.

The sequence identity can be easily calculated by known methods, including, but not limited to those described in Computational Molecular Biology, Lesk, A. N., ed., Oxford University Press, New York (1988), Biocomputing: Informatics and Genome Projects, Smith, D.W., ed.,Academic Press, New York (1993); Computer Analysis of Sequence Data, Parti, Griffin, A. M. e Griffin, H. G., eds., Humana Press, New Jersey (1994); Sequence Analysis in Molecular Biology, von Heinge, G., Academic Press (1987); Sequence Analysis Primer, Gribskov, M. and Devereux, J., eds., M. Stockton Press, New York (1991); and Carillo, H. and Lipman, D., SIAM J. Applied Math., 48: 1073 (1988), the teachings of which are herein incorporated by reference.

Preferred methods to determine the sequence identity are designated to provide the highest correspondence between the tested sequences. Methods for determining the sequence identity are encoded in publicly available computer programs that determine the sequence identity between given sequences. Examples of said programs includes but are not limited to the program packet GCG (Devereux, J., et al., NucleicAcids Research, 12(1):387 (1984)), BLASTP, BLASTN e FASTA (Altschul, S. F. et al., J. Molec. Biol., 215:403 - 410 (1990). The BLASTX program is publicly available at NCBI and other sources (BLAST Manual, Altschul, S. et al., NCVI NLM NIH Bethesda, MD 20894, Altschul, S. F. et al., J. Molec. Biol., 215:403 - 410 (1990), the teachings of which are herein incorporated by reference. These programs ideally align sequences using standard interval weights to produce the highest level of sequence identity between the given and reference sequences.

A person skilled in the art knows that the composition and the vaccine described herein can incorporate sterile injectable, physiologically acceptable known solutions.

In an additional embodiment, the present invention refers to a method to induce an immune response in mammal animals, particularly bovine cattle, wherein the method comprises the step of administering a therapeutically effective amount of the peptide combination, of the chimeric peptide or the immunogenic composition described herein, to an animal in need thereof

In an additional embodiment, the present invention refers to a kit, comprising the peptide combination, the chimeric peptide or the immunogenic composition, as defined by the present invention and instructions for use. Preferably, the kits are for vaccination.

the present invention is further illustrated by the following examples which, however, must not be interpreted as being limitations to the scope of the invention. The characteristics described in the above specification and in the following examples can, separately and in any combination, serve as basis for the execution of the present invention in its various forms of embodiment and possible execution.

## EXAMPLES

### EXAMPLE 1. IMMUNE RESPONSE INDUCTION IN BOVINES AND EVALUATION OF DEGREE OF TICK INFESTATION:

The proposal of the present invention has the purpose of evaluating the immunoprotection of peptides selected in immunized bovine cattle and, subsequently, challenged by means of artificial infestation with larvae of *R. microplus* ticks. The peptides used were divided in groups, as follows:

- **Group 1:** peptides of SEQ ID NO: 1 to 6; and
- **Group 2:** peptides of SEQ ID NO: 7 to 12.

The weaned animals of the Angus breed, with average weight around 250 kg, were grouped in number of 5 animals each, and submitted to vaccine stimulation using 200$\mu$g of peptides designed and associated to the KLH carrier, apart from being solubilized in adjuvant Montanide®.

The control group received only the inoculation of the adjuvant and saline solution in the same volume for the dose.

Each animal was stimulated with three doses, subcutaneously administered, in a volume of 2mL, in an interval of 21 days and, subsequently, submitted to the challenge with 15.000 larvae of the *Rhipichephalus microplus* tick, 21 days after the last inoculation.

After the collection of the ticks in the individual stalls, there were obtained the results of the infestation and performed the incubation of these to know their biological performance. The data were compared with the control group.

The animals were monitored regarding the clinical aspects without presenting alterations in the body temperature, feeding behavior and reaction at the place of the vaccine inoculation.

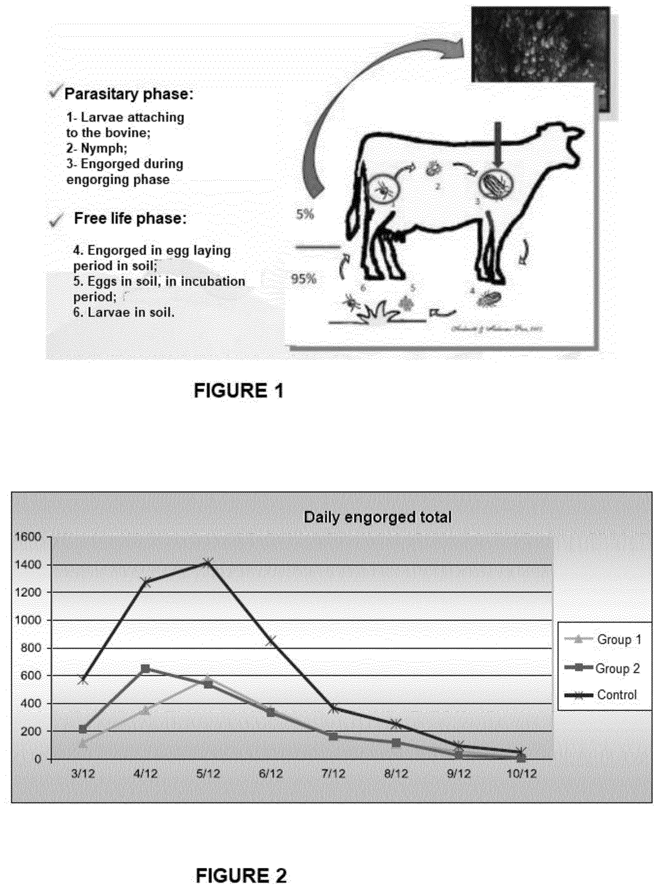

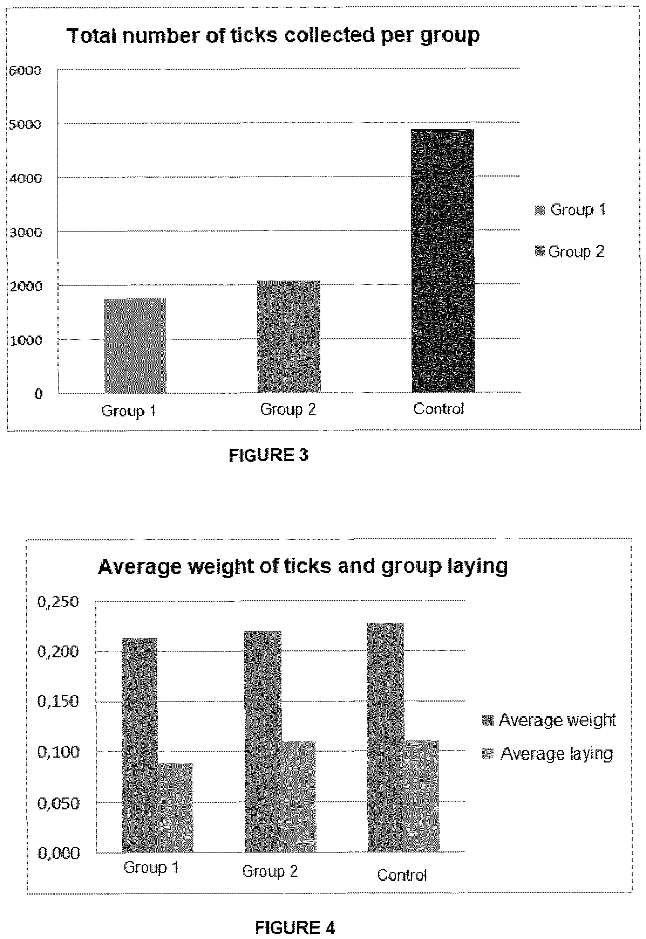

Thus, when carrying out the collection of ticks in the individual stalls, there were obtained results of the degree of infestation and subsequently of the tick incubation, compared with the control group, without vaccine. Figure 2 shows the average amount of ticks (engorged) collected daily by group, along the period of tick loss in the infested animals. The total number of ticks produced in groups 1 and 2 after the challenge is illustrated in Figure 3.

At the laboratory the average weight of the engorged females - engorged - collected daily during the experiment and the respective weight of the egg production, shown in Figure 4.

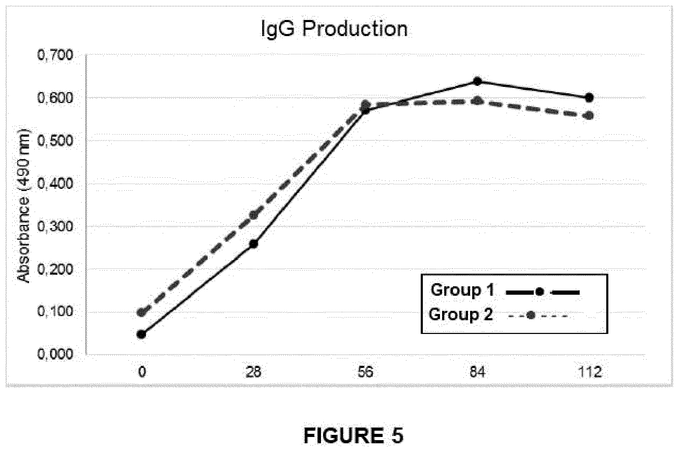

All the samples of the egg mass were incubated until the hatching of the larvae. Subsequently, the hatchability rate was evaluated for the purposes of calculation of the efficacy of the immunoprotection of the bovines. In Figure 5 we can observe the production of IgG in groups 1 and 2 which corresponds to the immune response of the animals in face of the peptide stimulation. The results were observed in the ELISA method.

**EXAMPLE 2. EVALUATION REGARDING VACCINE EFFICACY:**

The calculation of the efficacy involved the following parameters:

- Number of ticks;
- Weight of engorged ticks;
- Weight of the eggs; and
- Hatchability

For the calculation of the efficacy the following formula was used:

$$Ef = 100x\ [1-(NET\ x\ EW\ x\ EF\ x\ EC)]$$

In the above formula we have:

NET = total number of ticks of the immunized group/total number of ticks of the control group;

EW = (egg weight/number immunized ticks) / (egg weight/number control ticks);

EF = (egg weight/weight immunized ticks) / (egg weight/weight control ticks);

and

EC = % hatchability immunized larvae / % control hatchability.

The efficacy percentages, in comparative terms, were obtain according to the data and table below:
Group 1: NET = 0,3600; EW = 0,8489; EF = 0,9809; and EC= 1,0282.
Group 2: NET = 0,4221; EW = 1,0372; EF= 1,0861; and EC= 1,0356.

| Groups | Efficacy |
|---|---|
| 1 | 69 |
| 2 | 51 |

As can be observed, both Groups 1 and 2 presented very satisfactory results, and higher than the results observed in the state of the art

## Claims

1. **PEPTIDE COMBINATION characterized by** being of at least 2 immunogenic peptides selected from the group consisting in the peptides of SEQ ID NO:1 to 12.

2. **COMBINATION,** according to claim 1, **characterized by** being from 2 to 24 peptides,

3. **COMBINATION,** according to claim 1, **characterized by** being from 2 to 8 peptides

4. **COMBINATION,** according to claim 1, **characterized by** being from 3 to 7 peptides

5. **COMBINATION,** according to claim 1, **characterized by** being from 4 to 6 peptides.

6. **COMBINATION,** according to any one of claims 1 to 5, **characterized by** comprising 6 peptides selected from the group consisting in peptides of the SEQ ID NO: 1 to 12.

7. **COMBINATION,** according to claim 6, **characterized by** the 6 selected peptides being the peptides of the SEQ ID NO: 1 to 6.

8. **COMBINATION,** according to claim 6, **characterized by** the 6 selected peptides being the peptides of the SEQ ID NO: 7 to 12.

9. **CHIMERIC PEPTIDE, characterized by** comprising combinations of at least 2 immunogenic peptides selected from the group consisting in the peptides of SEQ ID NO: 1 to SEQ ID NO: 12, bonded between each other.

10. **CHIMERIC PEPTIDE,** according to claim 9, **characterized by** comprising combinations of 2 to 24 peptides.

11. **CHIMERIC PEPTIDE,** according to claim 9, **characterized by** comprising combinations of 2 to 8 peptides.

12. **CHIMERIC PEPTIDE,** according to claim 9, **characterized by** comprising combinations of 3 to 7 peptides.

13. **CHIMERIC PEPTIDE,** according to claim 9, **characterized by** comprising combinations of 4 to 6 peptides.

14. **CHIMERIC PEPTIDE,** according to any one of claims 9 to 13, **characterized by** comprising combination of 6 peptides selected from the group that consists in the peptides of SEQ ID NO: 1 to 12.

15. **CHIMERIC PEPTIDE,** according to claim 14, **characterized by** the 6 selected peptides being the peptides of SEQ ID NO: 1 to 6.

16. **CHIMERIC PEPTIDE,** according to claim 14, **characterized by** the 6 selected peptides being the peptides of SEQ ID NO: 7 to 12.

17. **IMMUNOGENIC COMPOSITION, characterized by** comprising the peptide combination as described in claims 1 to 8 and pharmaceutically acceptable excipients.

18. **IMMUNOGENIC COMPOSITION, characterized by** comprising the chimeric peptide as described in claims 9 to 16 and pharmaceutically acceptable excipients.

19. **IMMUNOGENIC COMPOSITION,** according to any one of claims 17 to 18, **characterized by** further comprising an additional biologically active substance or compound.

20. **IMMUNOGENIC COMPOSITION,** according to any one of claims 17 to 19, **characterized by** being in the form of solution, mixture, powder, granules, aerosol or freeze dried.

21. **IMMUNOGENIC COMPOSITION,** according to any one of claims 17 to 20, **characterized by** being a vaccine.

22. **USE OF THE PEPTIDE COMBINATION,** as described in claims 1 to 8, **characterized by** being in the preparation of a composition for immunizing, treating, protecting, attenuating and/or preventing tick infestations in mammals.

23. **USE OF THE PEPTIDE COMBINATION,** as described in claim 22, **characterized by** the mammal tick being Rhipichephalus microplus.

24. **USE OF THE PEPTIDE COMBINATION,** as described in claims 9 to 16, **characterized by** being in the preparation of a composition for immunizing, treating, protecting, attenuating and/or preventing mammal tick infestations.

25. **USE OF THE CHIMERIC PEPTIDE,** as described in claim 24, **characterized by** the mammal tick being Rhipichephalus microplus.

26. **USE OF A COMPOSITION,** as described in claims 17 to 21, **characterized by** being in the preparation of a medication for immunizing, treating, protecting, attenuating and/or preventing infestations by mammal ticks.

27. **USE OF A COMPOSITION,** as described in claim 26, **characterized by** the mammal tick being Rhipichephalus microplus.

28. **METHOD OF INDUCING AN IMMUNE RESPONSE, characterized by** comprising the steps of:

    a. Obtaining composition comprising therapeutically effective amount of the peptide combination of at least 2 immunogenic peptides selected from the group consisting in the peptides of SEQ ID NO: 1 to 12or of the chimeric peptide comprising combinations of at least 2 immunogenic peptides selected from the group consisting in the peptides of SEQ ID NO: 1 to SEQ ID NO: 12, bonded between each other,
    b. Administering the composition obtained in (a) to an animal;
    c. Monitoring the degree of tick infestation in animal of (b).

29. **METHOD,** according to claim 28, **characterized by** the composition obtained in (a) comprising peptide combination as described in any one of claims 1 to 8.

30. **METHOD,** according to claim 28, **characterized by** the composition obtained in (a) comprising chimeric peptide as described in any one of claims 9 to 16.

31. **METHOD,** according to claim 28, **characterized by** the composition obtained in (a) being an immunogenic composition as described in any one of claims 17 to 21.

32. **METHOD,** according to any one of claims 28 to 31, **characterized by** the composition obtained in (a) being a vaccine.

33. **METHOD,** according to any one of claims 28 to 32 **characterized by** the immune response being for immunizing, treating, protecting, attenuating and/or preventing tick infestations in a mammal animal, particularly bovine cattle.

34. **KIT characterized by** comprising the combination as described in claims 1 to 8 and instructions for use.

35. **KIT characterized by** comprising the chimeric peptide as described in claims 9 to 16 and instructions for use.

36. **KIT characterized by** comprising a composition as described in claims 17 to 21 and instructions for use.

**37. KIT** according to any one of claims 34 to 36, **characterized by** being for vaccination.

Parasitary phase:
1- Larvae attaching to the bovine;
2- Nymph;
3- Engorged during engorging phase
Free life phase:
4. Engorged in egg laying period in soil;
5. Eggs in soil, in incubation period;
6. Larvae in soil.
5%
95%

FIGURE 1

Daily engorged total
1600
1400
1200
1000
800
600
400
200
0
3/12
4/12
5/12
6/12
7/12
8/12
9/12
10/12
Group 1
Group 2
Control

FIGURE 2

Total number of ticks collected per group
6000
5000
4000
3000
2000
1000
0
Group 1
Group 2
Control
Group 1
Group 2

FIGURE 3

Average weight of ticks and group laying
0,250
0,200
0,150
0,100
0,050
0,000
Group 1
Group 2
Control
Average weight
Average laying

FIGURE 4

IgG Production
Absorbance (490 nm)
0,700
0,600
0,500
0,400
0,300
0,200
0,100
0,000
0
28
56
84
112
Group 1
Group 2

FIGURE 5

**INTERNATIONAL SEARCH REPORT**

International application No. **PCT/BR2022/050087**

**A. CLASSIFICATION OF SUBJECT MATTER**

C07K 19/00 (2006.01)i; A61K 39/00 (2006.01)i; A61K 38/10 (2006.01)i; A61K 38/17 (2006.01)i; A61P 33/14 (2006.01)

According to International Patent Classification (IPC) or to both national classification and IPC

**B. FIELDS SEARCHED**

Minimum documentation searched (classification system followed by classification symbols)

A61K 39/00

Documentation searched other than minimum documentation to the extent that such documents are included in the fields searched

Base de patentes do INPI-BR, Plataforma Lattes (http://lattes.cnpq.br/)

Electronic data base consulted during the international search (name of data base and, where practicable, search terms used)

GENESEQ, USGENE, WOGENE, GENBANK, DWPI

**C. DOCUMENTS CONSIDERED TO BE RELEVANT**

| Category* | Citation of document, with indication, where appropriate, of the relevant passages | Relevant to claim No. |
|---|---|---|
| A | GIACHETTO, P. F. et al. Gene Expression in the Salivary Gland of Rhipicephalus (Boophilus) microplus Fed on Tick-Susceptible and Tick-Resistant Hosts. Front Cell Infect Microbiol. 2020. Vol. 9:477. doi: 10.3389/fcimb.2019.00477. Abstract, Materials and Methods, Supplementary Material. | 1-27, 34-37 |
| A | ANDREOTTI, R. et al. Advances in tick vaccinology in Brazil: from gene expression to immunoprotection. Front Biosci (Schol Ed). 2018. Vol. 10, no. 1, pages: 127-142. doi: 10.2741/s504. Abstract, pages 130-132. | 1-27, 34-37 |
| A | AGUIRRE, A. A. R. et al. Design of the ATAQ peptide and its evaluation as an immunogen to develop a Rhipicephalus vaccine. Veterinary Parasitology. 2016. Vol. 221, pages: 30-38. https://doi.org/10.1016/j.vetpar.2016.02.032. Cited in the description. Abstract, section 3.1, figures 2 and 3. | 1-27, 34-37 |

☑ Further documents are listed in the continuation of Box C.  ☐ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 May 2022** | **25 May 2022** |

| Name and mailing address of the ISA/BR | Authorized officer |
|---|---|
| **National Institute of Industrial Property (Brazil)**<br>**Rua Mayrink Veiga, 9, 6º andar, CEP 20.090-910 Rio de Janeiro – RJ**<br>**Brazil** | **Flávia Riso Rocha** |
| Telephone No. **(55 21) 3037-3742, 3037-3984** | Telephone No. **55 21 30373493** |

Form PCT/ISA/210 (second sheet) (July 2019)

**INTERNATIONAL SEARCH REPORT**

International application No.
**PCT/BR2022/050087**

**C. DOCUMENTS CONSIDERED TO BE RELEVANT**

| Category* | Citation of document, with indication, where appropriate, of the relevant passages | Relevant to claim No. |
|---|---|---|
| A | CSORDAS, B. G. et al. Molecular characterization of the recombinant protein RmLTI-BmCG-LTB: Protective immunity against Rhipicephalus (Boophilus) microplus. PLoS One. 2018. Vol. 13, no. 2:e0191596. doi: 10.1371/journal.pone.0191596. Abstract, Materials and methods, Table 1. | 1-27, 34-37 |

Form PCT/ISA/210 (second sheet) (July 2019)

**INTERNATIONAL SEARCH REPORT**

International application No.
**PCT/BR2022/050087**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**

International application No.
**PCT/BR2022/050087**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **28-33**
because they relate to subject matter not required to be searched by this Authority, namely:

   Methods for treatment of the human or animal body by surgery or therapy, as well as diagnostic methods (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BLECHA, I.M.Z. et al.** Analysis of Bm86 conserved epitopes: is a global vaccine against Cattle Tick Rhipicephalus microplus possible?. *Braz. J. Vet. Parasitol., Jaboticabal,* 2018, vol. 27 (3), 267-279
- **AGUIRRE, A.A.R. et al.** Design of the ATAQ peptide and its evaluation as an immunogen to develop a Rhipicephalus vaccine. *Veterinary Parasitology,* 2016, vol. 221, 30-38
- **ALMAZÁN, C. et al.** Identification and characterization of Rhipicephalus (Boophilus) microplus candidate protective antigens for the control of cattle tick infestations. *Parasitol Res.,* 2010, vol. 106, 471-479
- **GENTILUCCI, L. ; DE MARCO, R. ; CERISOLI, L.** Chemical Modifications Designed to Improve Peptide Stability: Incorporation of Non-Natural Amino Acids, Pseudo-Peptide Bonds, and Cyclization. *Current Pharmaceutical Design,* 2010, vol. 16, 3185-3203
- **REMINGTON.** Remington: The Science and Practice of Pharmacy
- **ROWE.** Handbook of pharmaceutical excipients
- Computational Molecular Biology. Oxford University Press, 1988
- Biocomputing: Informatics and Genome Projects. Academic Press, 1993
- Computer Analysis of Sequence Data. Humana Press, 1994
- **VON HEINGE, G.** Sequence Analysis in Molecular Biology. Academic Press, 1987
- Sequence Analysis Primer. M. Stockton Press, 1991
- **CARILLO, H. ; LIPMAN, D.** *SIAM J. Applied Math.,* 1988, vol. 48, 1073
- **DEVEREUX, J. et al.** *NucleicAcids Research,* 1984, vol. 12 (1), 387
- **ALTSCHUL, S. F. et al.** *J. Molec. Biol.,* 1990, vol. 215, 403-410